# FASCICULE DE BREVET EUROPEEN

(11) **EP 1 933 743 B1**
(45) Date de publication et mention de la délivrance du brevet: **23.03.2011**
(21) Numéro de dépôt: 06831198.4
(22) Date de dépôt: 20.09.2006
(51) Int. Cl.: A61B 17/88

(54) **ANCILLAIRE DE MISE EN TENSION D'UN LIEN SOUPLE**
ZUBEHÖR ZUM SPANNEN EINES FLEXIBLEN VERBINDUNGSGLIEDS
ACCESSORY FOR TENSIONING A FLEXIBLE LINK

(30) Priorité: 21.09.2005 FR 0509629
(43) Date de publication de la demande: 25.06.2008
(73) Titulaire: ZIMMER SPINE, 33000 Bordeaux (FR)
(72) Inventeur: BACCELLI, Christian, F-33650 Saucats (FR); LE COUEDIC, Régis, F-78570 Andresy (FR); MAZDA, Keyvan, F-75020 Paris (FR)
(74) Mandataire: Besnard, Christophe Laurent
(86) Numéro de dépôt international: PCT/FR2006/050909
(87) Numéro de publication internationale: WO 2007/034112

(56) Documents cités:
- EP-A- 0 780 096
- WO-A-2004/010881
- US-A- 5 964 769
- US-A1- 2004 138 666

## Description

La présente invention a pour objet un ancillaire de mise en tension d'un lien souple servant à fixer un implant sur un élément osseux en formant une première boucle autour de l'élément osseux.

Le rachis est formé par la superposition de vertèbres, normalement alignées selon un axe vertébral, des lombaires jusqu'aux cervicales et chacune présentant une paroi postérieure de laquelle fait saillie l'apophyse épineuse et deux bords latéraux des parois desquels font saillies les côtes et/ou les apophyses transverses. Lorsque le rachis d'un individu présente une courbure anormale, les vertèbres sont inclinées les unes par rapport aux autres et par rapport audit axe vertébral. Les bords latéraux des vertèbres situés d'un même côté sont ainsi rapprochés les uns des autres et forment une concavité alors que les bords latéraux de l'autre côté apparaissent éloignés les uns des autres et forment une convexité.

Afin de redresser la colonne vertébrale, les bords latéraux des vertèbres du côté concave sont éloignés les uns des autres et portés, les uns par rapport aux autres, à une distance sensiblement équivalente à celle qui sépare les bords latéraux de l'autre côté. Pour maintenir ensuite les vertèbres les unes par rapport aux autres, des dispositifs connus comprennent des vis que l'on insère dans les vertèbres ou des crochets que l'on introduit le long de la paroi interne du canal rachidien et des tiges destinées à relier les vis ou les crochets.

Les crochets sont généralement introduits deux par deux dans chaque vertèbre et de chaque côté à proximité des pédicules, leur tête faisant saillie de la paroi postérieure de la vertèbre, une de chaque côté de l'apophyse épineuse. Les têtes formant tulipe, par exemple, sont susceptibles de recevoir une tige qui est bloquée au moyen d'un écrou vissé sur la tête et en appui sur la tige. Les rangées constituées par les têtes de crochet situées de chaque côté des apophyses épineuses sont reliées entre elles et maintenues en position fixe par deux tiges parallèles entre elles et à l'axe du rachis.

Cependant, l'utilisation de ces crochets est délicate puisque l'opérateur ne doit en aucun cas affecter la moelle épinière qui s'étend au centre du canal rachidien, sous peine de provoquer une paralysie du patient.

L'utilisation des vis permet de diminuer les risques de l'intervention. Elles présentent également des têtes formant tulipe et sont insérées deux par deux sur la paroi postérieure des vertèbres dans les pédicules de chaque côté de l'apophyse épineuse. Ainsi, les vis constituent des points de fixation dans les vertèbres pour les maintenir les unes par rapport aux autres. Cependant, elles sont nécessairement introduites dans le pédicule des vertèbres qui, dans certaines circonstances, est de faible taille ou détérioré.

Un problème qui se pose et que vise à résoudre la présente invention est alors de constituer des points de fixation, lorsqu'il n'est pas possible d'introduire de vis dans les vertèbres de la portion incurvée et que l'utilisation des crochets s'avère trop dangereuse.

Dans la demande de brevet PCT WO2004/010881 au nom de la demanderesse, on a décrit un système de fixation vertébrale qui permet de résoudre ce problème.

Ce système de fixation vertébrale susceptible d'être monté sur une vertèbre du rachis pour la relier à une tige comprend :
- une pièce de liaison disposée au regard de ladite côte et/ou de ladite apophyse transverse, susceptible d'être reliée à ladite tige ;
- un lien flexible de forme allongée susceptible de relier ensemble ladite pièce de liaison et au moins une côte et/ou une apophyse transverse ; et
- des moyens de blocage réglables solidaires de ladite pièce de liaison, ledit lien présentant une première extrémité solidaire de ladite pièce de liaison et une seconde extrémité libre susceptible de coulisser dans ladite pièce de liaison en formant une boucle, lesdits moyens de blocage étant susceptibles de maintenir en position fixe, simultanément, ladite pièce de liaison par rapport à ladite tige et une portion dudit lien comprise entre lesdites extrémités étant susceptible d'être bloquée en translation par rapport à ladite pièce de liaison par lesdits moyens de blocage réglables, par quoi la boucle présente une longueur déterminée de façon à bloquer le déplacement relatif de ladite tige et de ladite vertèbre dans des directions opposées l'une de l'autre.

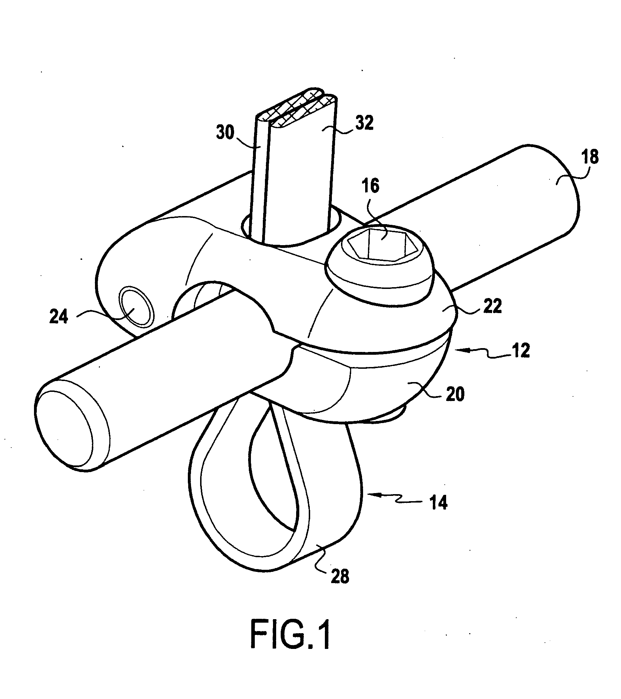

D'autres systèmes de fixation de vertèbre à lien souple peuvent être utilisés. C'est notamment le cas de celui qui est représenté sur la figure 1 annexée.

Il comprend une pièce de liaison 12 constituée par deux mâchoires 20 et 22 articulées à l'une de leur extrémité autour de l'axe 24. Ces deux mâchoires comportent des évidements permettant la mise en place d'une tige 18 et le passage d'une tresse ou lien 14, ce lien formant une boucle 28 d'un côté de la pièce de liaison 12 et deux extrémités libres 30 et 32 de l'autre côté de cette pièce. Le système de liaison comprend également un organe de blocage constitué par la vis 16 qui peut être engagée dans les extrémités des mâchoires 20 et 22 opposées à leur extrémité articulée. Les portions du lien 14 engagées dans les évidements sont rendues solidaires de la pièce de liaison par coincement entre les parois des évidements de la pièce de liaison et la tige 18 lorsque la vis de blocage 16 est complètement vissée.

On comprend que pour que cet ensemble soit correctement fixé sur une apophyse transverse sur une côte ou sur une partie de l'arc postérieur d'une vertèbre, il est nécessaire d'exercer une tension sur les extrémités libres 30 et 32 du lien 14.

On comprend également que dans le cas du premier système de fixation décrit, il est également nécessaire d'exercer une tension sur l'unique extrémité libre du lien pour assurer une fixation correcte sur l'élément osseux.

On connaît par le brevet US 5,964,769 un dispositif servant à exercer une tension sur un câble utilisé pour fixer un dispositif médical sur un os. Ce dispositif présente les inconvénients d'agir directement sur le dispositif de serrage du lien et aucune disposition ne permet de contrôler la tension exercée sur le lien.

Un objet de la présente invention est de fournir un ancillaire de mise en tension d'un lien souple d'un implant qui assure une mise sous tension efficace tout en étant d'un emploi aisé pour le chirurgien.

Pour atteindre ce but, selon l'invention, l'ancillaire de mise en tension d'un lien souple servant à fixer un implant sur un élément osseux d'un patient en formant une première boucle autour de l'élément osseux, ledit lien présentant au moins une extrémité faisant saillie hors dudit implant, se caractérise en ce qu'il comprend :
- une tige ayant une première extrémité munie de moyens d'appui sur ledit implant ;
- une pièce mobile en translation entourant ladite tige sur une partie de sa longueur ;
- un moyen d'accrochage de l'extrémité dudit lien, ledit moyen d'accrochage étant relié à ladite pièce mobile par un système dynamométrique ; et
- des moyens de commande montés sur ladite pièce mobile pour provoquer un déplacement de la pièce mobile par rapport à ladite tige tendant à écarter la première extrémité de la tige par rapport à ladite pièce mobile par quoi une tension est exercée sur ledit lien par rapport audit implant.

On comprend que la tige étant en appui sur l'implant, la pièce mobile sur laquelle est accroché soit la deuxième boucle du lien, soit l'extrémité libre de ce lien permet d'exercer une tension sur ce lien assurant ainsi un serrage convenable de la première boucle du lien sur l'élément osseux.

En outre, lorsque le chirurgien agit sur les moyens de commande, celui-ci sait à quel moment la tension convenable est appliquée, ce qui permet d'éviter une rupture intempestive du lien ou une détérioration de l'élément osseux.

De préférence, l'ancillaire comprend en outre un système anti-retour pour solidariser temporairement en translation la tige et la pièce mobile en l'absence d'action sur les moyens de commande.

De préférence, le système dynamométrique comprend un chariot mobile en translation par rapport à la tige et à la pièce mobile, ledit ergot d'accrochage du lien étant solidaire dudit chariot et un ressort de compression interposé entre ledit chariot et une partie de la pièce mobile.

De préférence également, les moyens de commande comprennent une gâchette montée pivotante par rapport à la pièce mobile et présentant une partie d'actionnement manuelle et un doigt agissant sur ladite tige.

De préférence encore, l'ancillaire comprend une poignée solidaire de la pièce mobile disposée de telle manière que l'utilisateur puisse saisir simultanément ladite gâchette et ladite poignée.

D'autres caractéristiques et avantages de l'invention apparaîtront mieux à la lecture de la description qui suit d'un mode de réalisation de l'invention donné à titre d'exemple non limitatif. La description se réfère aux figures annexées sur lesquelles :
- la figure 1 déjà décrite est un exemple d'implant à lien de fixation avec lequel l'ancillaire selon l'invention peut être avantageusement utilisé ;
- la figure 2 est une vue en perspective de l'ensemble de l'ancillaire selon l'invention ;
- la figure 3 est une vue éclatée de l'ancillaire démonté montrant les mécanismes internes ; et
- la figure 4 est une vue en perspective montrant l'utilisation de l'ancillaire avec un implant du type représenté sur la figure 1.

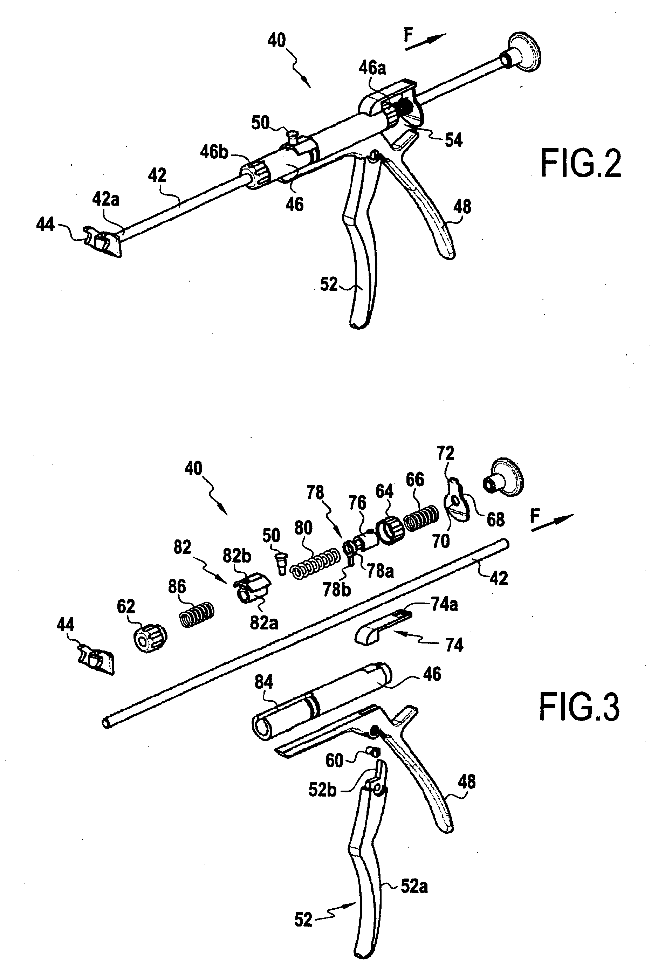

En se référant tout d'abord à la figure 2, on va décrire l'ensemble de l'ancillaire 40. Celui-ci est essentiellement constitué par une tige 42 dont la première extrémité 42a est équipée de moyens d'appui 44 sur l'implant dont on veut tendre le lien. L'ancillaire 40 comporte également une pièce mobile 46 qui est mobile en translation par rapport à la tige 42. Cette pièce mobile 46 de forme générale cylindrique est munie d'une poignée 48. La pièce mobile 46 comporte également un ergot 50 sur sa partie opposée à la poignée 48. Comme on l'expliquera ultérieurement, l'ergot 50 sert à accrocher le lien auquel on veut appliquer une tension. L'ancillaire 40 comporte aussi un organe de commande constitué par une gâchette 52. Ainsi qu'on l'expliquera ultérieurement, l'actionnement de la gâchette 52 permet de provoquer un mouvement de recul selon la flèche F de la pièce mobile 46 par rapport à la tige 42. En outre, à son extrémité 46a opposée à son extrémité 46b la plus proche de l'élément d'appui 44, la pièce mobile 46 est équipée d'un système anti-retour agissant sur la tige 42. Comme on l'expliquera plus en détail ultérieurement, le système anti-retour 54 permet de solidariser temporairement en translation la tige 42 et la pièce mobile 46 lorsque aucune action n'est exercée sur la gâchette 52.

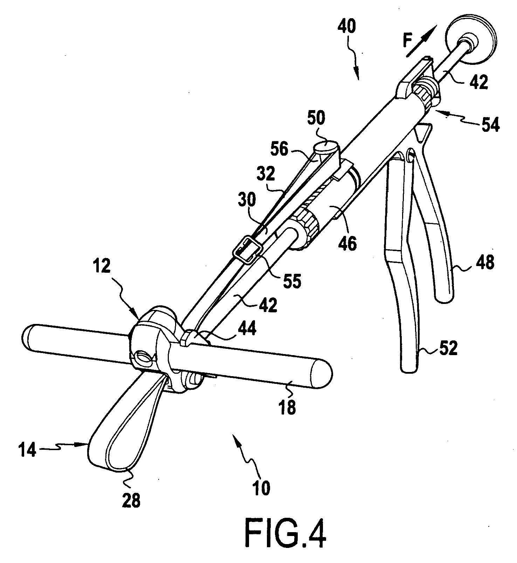

En se référant maintenant plus particulièrement à la figure 4, on va expliquer de façon générale, l'utilisation de l'ancillaire 40. Sur cette figure, on a représenté un système de fixation vertébral 10 du type représenté sur la figure 1. Sur cette figure, on voit la tige 18, la pièce de liaison 12 et la première boucle 28 de fixation formée par le lien 14 du système de fixation. Sur cette figure, on voit également que l'extrémité libre 32 du lien 14 est reliée à l'autre extrémité libre 30 de ce même lien par un élément de solidarisation d'un type convenable 55. Ainsi, le lien 14 forme une deuxième boucle 56.

En utilisation, les moyens d'appui 44 de l'ancillaire sont en appui sur la tige 18 de part et d'autre de la pièce de liaison 12. La deuxième boucle 56 du lien 14 est engagée sur l'ergot 50 de la pièce mobile 46 de l'ancillaire. On comprend que lorsque le chirurgien exerce une action en se servant de la gâchette 52 et de la poignée 48, celui-ci obtient un mouvement de recul selon la direction F de la pièce mobile 46 par rapport à la tige 42, ce qui provoque une action de traction sur l'ensemble du lien et en particulier sur la boucle 28 de celui-ci. Le chirurgien peut exercer des actions successives à l'aide de la gâchette 52 grâce à la présence du système anti-retour 54. Comme on l'expliquera ultérieurement, de préférence, l'ancillaire est également équipé d'un système dynamométrique qui permet au chirurgien de voir lorsqu'une tension convenable a été exercée sur le lien 14. Lorsque la tension convenable a été exercée, l'ancillaire 40 est dégagé de la boucle 56 du lien 14 et les parties du lien 14 dépassant de la pièce de liaison 12 sont sectionnées.

En se référant maintenant à la figure 3, on va décrire plus en détails les mécanismes de l'ancillaire 40. Sur cette figure, on retrouve la pièce mobile 46 de forme cylindrique sur laquelle est fixée la poignée 48. La gâchette 52 est articulée par rapport à la poignée 48 autour d'un axe 60. La gâchette 52 comporte une partie de préhension 52a et un doigt de commande de la tige 52b disposé au-delà de l'axe de pivotement 60. Le doigt 52b pénètre dans la pièce mobile 46 par un évidement non représenté. La tige 42 est engagée à coulissement dans la pièce mobile 46.

Différents éléments qui vont être décrits maintenant sont montés autour de la tige 42 à l'intérieur de la pièce mobile 46 dont les extrémités ouvertes sont obturées par des embouts 62 et 64 percés d'un alésage axial pour permettre le passage de la tige 42. A l'extérieur de la pièce mobile 46, est monté autour de la tige 42 un ressort 66 et une plaquette 68 constituant le système anti-retour. La plaquette 68 est percée d'un alésage 70 dont le diamètre est légèrement supérieur à celui de la tige 42. La plaquette 68 comporte une languette 72 qui peut venir en appui sur un bras 74 solidaire de la partie arrière de la pièce mobile 46. A l'intérieur de la pièce mobile 48 et en partant de son extrémité obturée par l'embout 64, on trouve un cylindre entretoise 76, une pièce de transmission 78 constituée par une partie annulaire 78a et par un ergot 78b apte à coopérer avec le doigt 52b de la gâchette 52. A la pièce de transmission 78 est associé le ressort 80. On trouve ensuite un chariot 82 comportant une portion cylindrique 82a engagée autour de la tige 42 et deux ailes externes 82b. Les ailes 82b du chariot 82 sont externes à la pièce mobile 46 grâce à la fente longitudinale 84 que présente cette dernière. Sur la partie externe du chariot 82 est fixé l'ergot d'accrochage 50. Au chariot 82 est associé un ressort dynamométrique 86 qui est interposé entre la face avant du chariot de la partie cylindrique 82a du chariot 82 et l'embout 62

En l'absence d'action sur la gâchette 52, la plaquette 68 du système anti-retour est inclinée par rapport à la tige 42 en raison de la présence de l'extrémité 74a du bras 74 entraînant ainsi la solidarisation temporaire en translation de la tige 42 et de la pièce mobile 46. Lorsqu'on exerce une action sur la gâchette 52, le déplacement de la tige libère la plaquette 68 et autorise ainsi le déplacement relatif de la tige 42 par rapport à la pièce mobile 46. De la même manière, en l'absence d'action sur la gâchette 52, la pièce de transmission 78 est libre, en revanche, lorsqu'on agit sur la gâchette 52, le doigt 52b agit sur l'ergot 78b de la pièce de transmission 78, ce qui entraîne la solidarisation temporaire de celle-ci et de la tige 42. Cette solidarisation temporaire permet le déplacement de la tige 42 par rapport à la pièce 46 sous l'effet de l'actionnement de la gâchette.

En ce qui concerne le système dynamométrique, son fonctionnement est simple. Sous l'effet du déplacement de la tige 42 selon la direction F par rapport à la pièce 46, le ressort dynamométrique 86 se comprime entraînant le déplacement relatif du chariot 82. Un repère ménagé sur la face externe de la pièce mobile 46 permet de détecter que la tension convenable a été appliquée, cette tension correspondant bien entendu à une compression prédéterminée du ressort dynamométrique 86.

Dans la description précédente, on a envisagé le cas où le lien 14 comportait une deuxième boucle qui servait à l'accrochage sur l'ergot de tension 50 de l'ancillaire. Dans le cas où le lien de l'implant ne comporte qu'une extrémité libre, cette extrémité peut être accrochée sur l'ergot 50 ou sur tout autre système d'accrochage convenable pour exercer de la même manière la tension souhaitée sur l'extrémité du lien et donc sur la boucle 28 que forme ce lien.

## Revendications

1. Ancillaire de mise en tension d'un lien souple (14) servant à fixer un implant sur un élément osseux d'un patient en formant une première boucle autour de l'élément osseux, ledit lien présentant au moins une extrémité faisant saillie hors dudit implant, ledit ancillaire **se caractérisant en ce qu'**il comprend :
. une tige (42) ayant une première extrémité munie de moyens d'appui (44) sur ledit implant ;
. une pièce mobile en translation (46) entourant ladite tige (42) sur une partie de sa longueur ;
. un moyen d'accrochage (50) de l'extrémité dudit lien, ledit moyen d'accrochage (50) étant relié à ladite pièce mobile par un système dynamométrique (82, 86) ; et
. des moyens de commande (52) montés sur ladite pièce mobile (46) pour provoquer un déplacement de la pièce mobile par rapport à ladite tige tendant à écarter la première extrémité (42a) de la tige par rapport à ladite pièce mobile par quoi une tension est exercée sur ledit lien par rapport audit implant.

2. Ancillaire selon la revendication 1, **caractérisé en ce qu'**il comprend en outre un système anti-retour (68) pour solidariser temporairement en translation ladite tige (42) et ladite pièce mobile (46) en l'absence d'action sur les moyens de commande (52).

3. Ancillaire selon l'une quelconque des revendications 1 et 2, **caractérisé en ce que** le lien (14) comporte deux extrémités raccordées entre elles pour former une deuxième boucle (56).

4. Ancillaire selon la revendication 3, **caractérisé en ce que** les moyens d'accrochage du lien comprennent un ergot (50).

5. Ancillaire selon l'une quelconque des revendications 1 à4, **caractérisé en ce que** ledit système dynamométrique comprend un chariot (82) mobile en translation par rapport à la tige (42) et à la pièce mobile (46), ledit moyen d'accrochage (50) du lien étant solidaire dudit chariot, et un ressort de compression (86) interposé entre ledit chariot et une partie de ladite pièce mobile.

6. Ancillaire selon l'une quelconque des revendications 1 à 5, **caractérisé en ce que** lesdits moyens de commande comprennent une gâchette (52) montée pivotante par rapport à la pièce mobile (46) et présentant une partie d'actionnement manuel (52a) et un doigt (52b) agissant sur ladite tige (42).

7. Ancillaire selon la revendication 6, **caractérisé en ce qu'**il comprend en outre une poignée (48) solidaire de ladite pièce mobile (46) disposée de telle manière que l'utilisateur puisse saisir simultanément ladite gâchette (52) et ladite poignée (48).

8. Ancillaire selon l'une quelconque des revendications 6 et 7, **caractérisé en ce que** lesdits moyens de commande comprennent en outre une pièce de transmission (78) constituée par une partie annulaire (78a) entourant ladite tige (42) et un ergot (78b) apte à coopérer avec ledit doigt (52b) de la gâchette (52).

## Claims

1. An instrument for tensioning a flexible tie (14) used for fastening an implant onto a bony element of a patient by forming a first loop around the bony element, said tie presenting at least one end that projects out from said implant, said instrument being **characterized in that** it comprises:
· a rod (42) having a first end provided with bearing means (44) for bearing against said implant;
· a moving part (46) that is movable in translation and that surrounds said rod (42) over a fraction of its length;
· holder means (50) for holding the end of said tie, said holder means (50) being connected to said moving part by a dynamometer system (82, 86); and
· control means (52) mounted on said moving part (46) to cause the moving part to move relative to said rod, thereby tending to move the first end (42a) of the rod away from said moving part, thereby exerting tension on said tie relative to said implant.

2. An instrument according to claim 1, **characterized in that** it further comprises an anti-return system (68) for temporarily preventing said rod (42) and said moving part (46) from moving in translation relative to each other, in the absence of action on the control means (52).

3. An instrument according to claim 1 or claim 2, **characterized in that** the tie (14) has two ends connected together to form a second loop (56).

4. An instrument according to claim 3, **characterized in that** the tie-holder means comprise a stud (50).

5. An instrument according to any one of claims 1 to 4, **characterized in that** said dynamometer system comprises a carriage (82) that is movable in translation relative to the rod (42) and to the moving part (46), said tie-holder means (50) being secured to said carriage, and a compression spring (86) interposed between said carriage and a portion of said moving part.

6. An instrument according to any one of claims 1 to 5, **characterized in that** said control means comprise a trigger (52) mounted to pivot relative to the moving part (46) and presenting a manual actuator portion (52a) and a finger (52b) that acts on said rod (42).

7. An instrument according to claim 6, **characterized in that** it further comprises a handle (48) secured to said moving part (46) and disposed in such a manner that the user can grasp said trigger (52) and said handle (48) simultaneously.

8. An instrument according to claim 6 or claim 7, **characterized in that** said control means further comprise a transmission part (78) constituted by an annular portion (78a) surrounding said rod (42) and a stud (78b) suitable for co-operating with said finger (52b) of the trigger (52).

## Patentansprüche

1. Zubehör zum Spannen eines flexiblen Verbindungsglieds (14), das dazu dient, ein Implantat auf einem Knochenelement eines Patienten zu befestigten, indem es eine erste Schlaufe um das Knochenelement bildet, wobei das Verbindungsglied mindestens ein Ende aufweist, das aus dem Implantat herausragt, wobei das Zubehör **dadurch gekennzeichnet ist, daß** es umfaßt:
- einen Stift (42) der ein erstes Ende hat, das mit Mitteln zur Auflage (44) auf dem Implantat versehen ist,
- ein translatorisch bewegbares Teil (46), das den Stift (42) auf einem Teil seiner Länge umgibt,
- ein Mittel zum Befestigen (50) des Endes des Verbindungsglieds, wobei das Mittel zum Befestigen (50) mit dem bewegbaren Teil durch ein dynamometrisches System (82, 86) verbunden ist, und
- Mittel zur Steuerung (52), die auf dem bewegbaren Teil (46) montiert sind, um eine Verschiebung des bewegbaren Teils bezogen auf den Stift, der dazu neigt, das erste Ende (42a) des Stifts bezogen auf das bewegbare Teil wegzuschieben, hervorzurufen, wodurch eine Spannung auf das Verbindungsglied bezogen auf das Implantat ausgeübt wird.

2. Zubehör nach Anspruch 1, **dadurch gekennzeichnet, daß** es ferner ein Rücklaufsicherungssystem (68) umfaßt, um den Stift (42) und das bewegliche Teil (46) in Abwesenheit von Einwirkung auf die Mittel zur Steuerung (52) vorübergehend gegen Translation zu befestigen.

3. Zubehör nach einem der Ansprüche 1 und 2, **dadurch gekennzeichnet, daß** das Verbindungsglied (14) zwei Enden beinhaltet, die miteinander verbunden sind, um eine zweite Schlaufe (56) zu bilden.

4. Zubehör nach Anspruch 3, **dadurch gekennzeichnet, daß** die Mittel zum Befestigen des Verbindungsglieds einen Zapfen (50) umfassen.

5. Zubehör nach einem der Ansprüche 1 bis 4, **dadurch gekennzeichnet, daß** das dynamometrische System einen bezogen auf den Stift (42) und das bewegbare Teil (46) translatorisch bewegbaren Wagen (82), wobei der Befestigungszapfen (50) des Verbindungsglieds an dem Wagen befestigt ist, und eine Druckfeder (86) umfaßt, die zwischen dem Wagen und einem Teil des bewegbaren Teils angebracht ist.

6. Zubehör nach einem der Ansprüche 1 bis 5, **dadurch gekennzeichnet, daß** die Mittel zur Steuerung einen Drücker (52) umfassen, der bezogen auf das bewegbare Teil (46) schwenkbar montiert ist und ein Teil zum manuellen Betätigen (52a) und einen Finger (52b) aufweist, der auf den Stift (42) wirkt.

7. Zubehör nach Anspruch 6, **dadurch gekennzeichnet, daß** es ferner einen Griff (48) umfaßt, der an dem beweglichen Teil (46) befestigt und so angeordnet ist, daß der Anwender den Drücker (52) und den Griff (48) gleichzeitig ergreifen kann.

8. Zubehör nach einem der Ansprüche 6 und 7, **dadurch gekennzeichnet, daß** die Mittel zur Steuerung ferner ein Kraftübertragungsteil (78) umfassen, das aus einem ringförmigen Teil (78a), das den Stift (42) umgibt, und einem Zapfen (78b) besteht, der in der Lage ist, mit dem Finger (52b) des Drückers (52) zusammenzuwirken.
